# EUROPEAN PATENT APPLICATION

(11) **EP 2 779 000 A1**
(43) Date of publication of application: **17.09.2014**
(21) Application number: 14158900.2
(22) Date of filing: 11.03.2014
(51) Int. Cl.: G06F 19/00, G06Q 30/00

(54) **Toolkit system and method for managing delivery of services and content for health care orofessionals via an electronic health record system**

(30) Priority: 14.03.2013 US 201361781727 P; 05.03.2014 US 201414197268
(71) Applicant: Physicians Interactive, Inc., Reading, MA 01867 (US)
(72) Inventor: Cassidy, Dotty, Mundelein, Illinois 6006 (US); Kochar, Dinesh, Grayslake, Illinois 60030 (US)
(74) Representative: Wilson Gunn

(57) **Abstract**

A system and method for managing the delivery of services such as drug coupons, medical messaging and patient adherence content to healthcare providers at the point of care providing quick access to information without interrupting workflow. The toolkit manager is integrated into the healthcare provider's electronic medical records/electronic health records system.

## Description

This is a nonprovisional patent application claiming priority of provisional application for patent No. 61/781,727, filed March 14, 2013, the complete subject matter of which is incorporated herein by reference in its entirety.

### BACKGROUND OF THE INVENTION

### FIELD OF THE INVENTION

This invention relates to a system and method for managing the delivery of services and content such as eCoupons and vouchers or drug brand messaging to healthcare professionals (HCP) within their electronic health records (EHR) system and/or electronic prescribing system at the point of care, and in particular, to provide quick access to information without interrupting the HCP or prescriber's workflow.

### DESCRIPTION OF RELATED ART

Pharmaceutical companies currently do not have a single integrated (with EHR) source to provide all their relevant resources to HCPs at the point of care with an EHR and/or ePrescribe system. There is no direct or continuous electronic way of communication between HCP and patient regarding adherence after the prescription is written that is integrated within EHR. Also, the EHR providers do not have an easy way to provide HCP access to drug specific eCoupons/Vouchers/Patient Education material and other resources.

U.S. Patent Application Publication No. US 2002/0032582 published March 14, 2002 discloses a method and system for dispensing medication and integrated data management which can include a medical office system with at least one computer which can be linked to at least one server and a central system through a network. The system includes one or more dispensers configured to controllably release a product in response to a control signal, an admission subsystem configured to maintain patient information and a prescription subsystem coupled to the one or more dispensers and configured to receive entry of prescription information. However, dispensing physical medication or drug samples requires a secure storage facility in the healthcare provider's office and management of stored samples usually obtained from meeting with a drug manufacturer sales representative. All these activities are time consuming and costly for a medical office.

U.S. Patent No. 8,121,868 issued February 21, 2012 disclosed systems and methods for providing an inducement to purchase medications incident to a physician's prescription for medication. The systems and methods generally provide a "content" such as a coupon for medicating patient's condition, etc. prior to the Point of Sale (POS) of the actual prescription to allow patients to review the content and possibly act on it prior to actually obtaining the medication. The content may be provided by a physician at or around the time of prescribing. However, the system manager does not provide any significant value added content or information about the medication or patient condition to a prescriber or healthcare provider. Such contents could educate or inform the prescriber about the medication and disease state and provide different ways to provide relevant content to the patient.

U.S. Patent No. 8,341,015 issued December 25, 2012, discloses a virtual sample cabinet system and method for prescription drug marketing by providing functionality for doctors and staff to remotely electronically download a "virtual sample cabinet" software program from a central server onto their desktop computers or mobile technology devices to allow them to centrally search, review product information and insurance coverage, print or electronically send prescription drug sample vouchers and co-pay savings coupons to selected pharmacies. However, this virtual sample cabinet (VSC) system and method for prescription drug marketing requires a prescriber or HCP to intervene with another separate system other than an electronic health record (EHR) application. The VSC system requires the prescriber to invest extra time to use an external system which should be achieved within the workflow of prescribing the drug. Further, the VSC system does not allow offering content or medical information based on the prescriber's specialty.

U.S. Patent No. 5,737,539 issued April 7, 1998 discloses an electronic prescription creation system which contains information about drug, formulations, patient history information and writing the prescription for patient at point of care. This can be referred to as Electronic Health Record application, but does not provide medication coupons or vouchers.

U.S. Patent No. 7,438,228 issued October 21, 2008, discloses systems for managing prescription of patient based on assessment on central server which consist of flow of determined medication, prescriber prescribe medication, send prescription to pharmacy, pharmacy fills the prescription- all of which are similar to an EHR workflow system.

U.S. Patent Application Publication No. 2012/0109689 published May 3, 2012 discloses a support system for receiving information and patient biometrics to update medical records and to improve quality of care and enhance the efficiency of operation of healthcare facilities and providers. When front-line healthcare doctors and nurses make various clinical decisions, MEGICS management system can provide them with relevant clinical knowledge in a timely manner. The healthcare management system has a framework that includes a business layer, a communication layer, a data layer, and a healthcare adapter which is designed to establish electrical and data communication with a hospital computer system. It has flexibility of presenting outputs in multiple formats, such as SML, HTMS, and TEXT and there will be very minimum risk for error in logic presentation, as it provides an Application Program Interface (API) Software Development Kit (SDK) through the network which can be integrated in any EHR system.

U.S. Patent Application Publication No. 2013/0041677 published February 14, 2013, discloses a computer-implemented cloud-based method and apparatus for providing an EHR offering to small medical practices. The system receives a request on a first computer to create a medical form. The medical form includes user selected fields corresponding to patient information. The system further receives signals on the first computer, where the signals describe form fields for the medical form. The system communicates the medical form from the first computer to the first tablet device for display on the first tablet device. The system receives form values, wherein at least some of the form values correspond to information about an appointment. Responsive to receiving a completion signal, the system generates a clinical narrative for the appointment using the completed form values. This system is an alternative to use of EHR, and its application by small medical groups allows doctors to communicate through dynamic medical forms containing patient information, prescription.

### SUMMARY OF THE INVENTION

Accordingly, it is therefore an object of this invention to provide a system and method for managing the delivery of services such as drug coupons, drug messaging and patient adherence to healthcare providers at the point of care via a medical and health records provider.

It is another object of this invention to provide a system and method for managing the delivery of other services to the healthcare professionals.

It is a further object of this invention to provide delivery of services not just to Electronic Health Record Systems but also to Hospitals, ACO, PBMs and other HCP Portals.

These and other objects are further accomplished by a toolkit manager system for managing delivery of services and content to a prescribing healthcare professional via an electronic health record provider comprising a server computing device including a memory and a processor, a database connected to the server computing device having stored therein pharmaceutical drug data and business rules, communication services located within the server computing device for interfacing with the electronic health record provider via web internet services, security means for providing authentication and authorization to verify that calls are received from the electronic health record provider that are allowed access, the server computing device verifies eligibility of a patient and the healthcare professional for a toolkit service using business rules data stored in the database, and the server computing device comprises executable instructions stored in the memory which when executed by the processor selects the toolkit services to be sent by the communication services to the electronic health record provider and the healthcare professional. The toolkit manager system comprises a toolkit manager for controlling access to services including electronic coupon, medication messaging and patient adherence, the toolkit manager including the server computing device and the database. The toolkit manager system comprises a toolkit manager having an input for directly receiving into the database pharmaceutical offerings on drugs. The toolkit manager system comprises a server computing device that generates a unique drug ID for each drug communicated with the electronic health record provider.

The objects are further accomplished by a computer-implemented method for managing delivery of services and content to a prescribing healthcare professional via an electronic health record provider comprising providing a server computing device including a memory and a processor, storing pharmaceutical drug and business rules in a database connected to the server computing device, providing communication services located within the server computing device for interfacing with the electronic health record provider via web internet services, testing authentication and authorization of the electronic health record provider via the web internet services, verifying eligibility of a patient and the healthcare professional for a toolkit service with the server computing device using business rules data stored in the database, and providing executable instructions stored in the memory of the server computing device which when executed by the processor selects the toolkit service to be sent by the communication services to the electronic health record provider and the healthcare professional. The method comprises the step of controlling access to services including electronic coupon, medication messaging and patient adherence using a toolkit manager having the server computing device and the database. The method comprises the step of providing the toolkit manager with an input for directly receiving pharmaceutical offerings/programs on drugs into the database. The method comprises the step of generating a unique drug ID by the server computing device for each drug communicated with the electronic health record provider.

Additional objects, features and advantages of the invention will become apparent to those skilled in the art upon consideration of the following detailed description of the preferred embodiment exemplifying the best mode of carrying out the invention as presently perceived.

### BRIEF DESCRIPTION OF THE DRAWINGS

The appended claims particularly point out and distinctly claim the subject matter of this invention. The various objects, advantages and novel features of this invention will be more fully apparent from a reading of the following detailed description in conjunction with the accompanying drawings in which like reference numerals refer to like parts, and in which:
Fig. 1 is a system block diagram of a toolkit manager interfacing with related systems according to the present invention.
Fig. 2 is a functional block diagram illustrating a high level system architecture of the toolkit manager system according to the present invention.
Fig. 3 is a toolkit manager workflow block diagram showing pharmaceutical steps and EHR provider steps.
Fig. 4 is a more detailed functional block diagram of the toolkit manager of the present invention.
Fig. 5 is a flow chart of a security method for authentication and authorization of service calls.
Fig. 6 is a block diagram of the drug setup process using a unique drug identifier.
Fig. 7 is a block diagram of Windows Communication Foundation (WCF) services operation interfacing with the toolkit database, business rules engine and logger.
Fig. 8 is a block diagram of a batch service operation interfacing with the toolkit database, entity framework, logger, and SMS and Email service business logic layer.
Fig. 9 is a flow chart of the business flow between an EHR provider and the toolkit manager of the present invention.
Fig. 10 is a block diagram of a one hub embodiment for the toolkit manager.
Fig 11 is a block diagram of a second hub embodiment for the toolkit manager.

### DESCRIPTION OF ILLUSTRATIVE EMBODIMENT

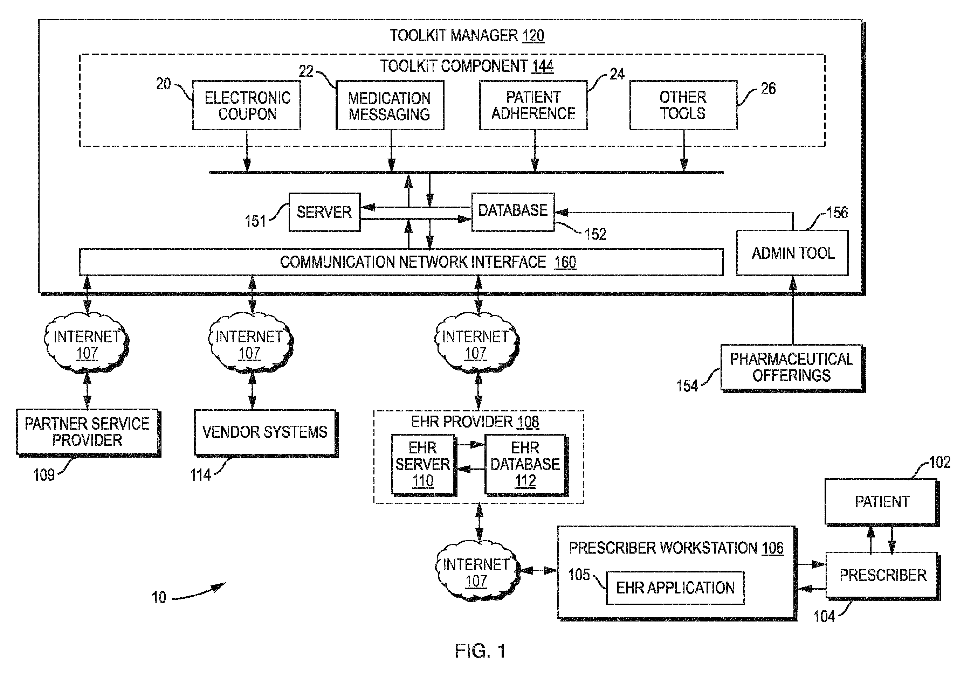

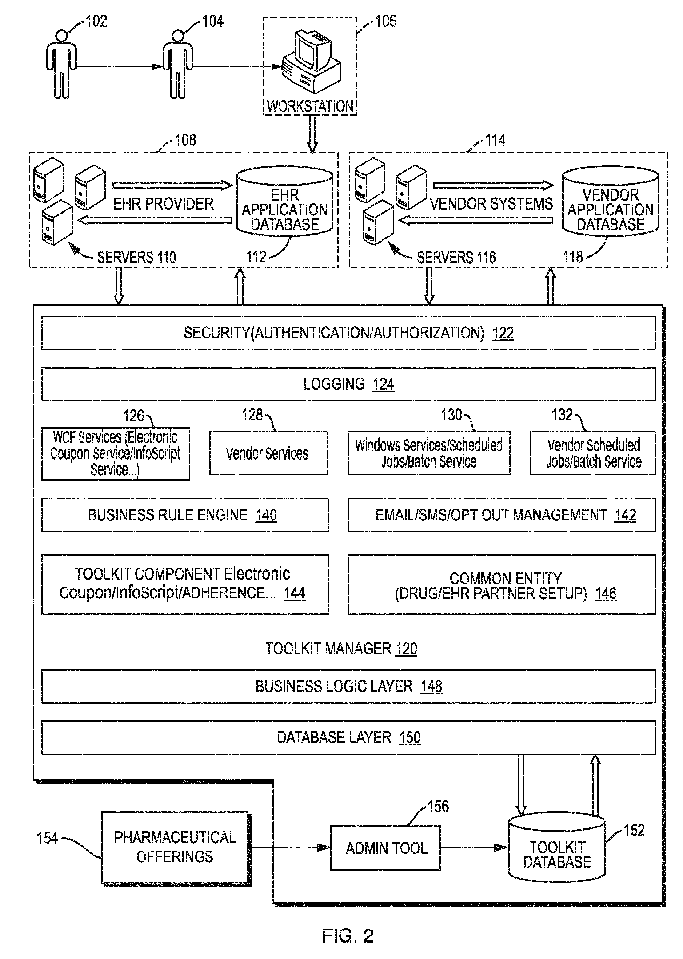

Referring to Fig. 1 and Fig. 2, Fig. 1 is a system block diagram of a toolkit manager (TM) 120 interfacing with related systems 10, via communications networks including the internet 107, an EHR provider 108, a vendor systems 114, a partner service provider 109 and pharmaceutical offerings 154 according to the present invention. Pharmaceutical Offerings 154 of drug coupons or vouchers and medication messaging are provided directly to the toolkit manager database 152 via an internal admin tool 156. Fig. 2 shows a functional block diagram of a toolkit manager 120 illustrating the system architecture of the toolkit manager 120 which manages the delivery of toolkit components 144 and services 126 and valuable information content via an EHR Provider 108 to a Healthcare Professional (HCP) 104 provided at the point of care with a patient 102. The toolkit manager 120 interfaces and operates with the electronic health record (EHR) system 108 (or electronic medical record (EMR) provider or electronic prescription system). The toolkit manager 120 is a single platform for all the EHR providers 108 to gain access to different offerings to patients 102 by seamlessly integrating within the workflow of EHR applications by calling a web service through a secure mechanism. The objective of the single platform is to deliver the services and content to the Prescriber or Healthcare Professional 104 at the point of care. The toolkit services 126 in Fig. 2 include providing patient coupons and vouchers for drug discounts via an electronic coupon component 20, providing medication messaging or Infoscript solution component 22 and/or providing a patient adherence component 24 for guidance and information.

The toolkit manager 120 comprises a computer server 151, database 152, communication network interface 160 and Admin Tool 156. The computer server 151 may be embodied by any standard server such as those widely available from Dell, Hewlett Packard or IBM and the database 152 may be embodied by a standard database such as those widely available from Microsoft, Oracle or IBM. The Microsoft SQL Server is used in the present invention. The communication network interface 160 includes standard communication software such as _WCF services 126 (HTTP, HTTPS, SOAP and XML) and Vendor Services 128 which provides a method of communications between Toolkit Manager 120 and third party systems like Partner Service Provider 109, Vendor Systems 114 and EHR Provider 108 over the World Wide Web (internet) 107, and a security engine 122 for performing authentication and authorization functions. The Admin Tool 156 is provided in the toolkit manager 120 to allow a user 204 to view or enter information such as pharmaceutical offerings 154, or other project/brand/drug/unique drug information into the toolkit database 152 through a separate internal port. The Admin Tool 156 provides the ability for its user to run and view a report for any program/offering across different toolkit components 144. The Admin Tool 156 includes web pages which are developed on Microsoft.net technology and these pages are hosted on server 151. The web pages are accessible through secure credentials by authorized user to which access has been granted. The user can perform configuration, setup, add/delete/update records search of pharmaceutical contacts, offerings, drugs, and brand. It also provides capability to generate analytical reports of pharmaceutical offerings.

Referring to Fig. 1 and Fig. 2, the EHR provider 108 comprises servers 110 and an EHR application database 112, and may be embodied by EHR systems such as those provided by Allscripts Healthcare, LLC or Greenway Medical Technologies, Inc. The HCP or prescriber 104 communicates with the EHR provider 108 via a prescriber workstation 106. The terms "HCP" and "prescribers" as referred to herein include, but are not limited to, physicians; physician assistants; certified registered nurse practitioners; advanced registered nurse practitioners; and other licensed professionals authorized to prescribe medications. The EHR provider 108 is a vendor which provides an EHR/EMR application to the HCP 104 to prescribe the medication for a patient 102. Electronic health records cover a wide scope of business functions and interact with a wide variety of health applications and service providers to provide different offerings on branded drugs to the patient based on business rules dictated by pharmaceutical companies. Some of the EHR applications which may be using the toolkit manager 120 to facilitate different services/offerings to a patient 102 within the workflow of the EHR application includes ePrescribe/EHR/EMR website, EHR/EMR client server applications and EHR/EMR mobility applications. The toolkit manager 120 interfaces with other vendor systems 114 via the internet 107 who want to utilize the toolkit manager 120 for offering a program for a given toolkit component 144 such as the electronic coupon component 20, medication messaging component 22, or patient adherence component 24 to its customers. The vendor systems 114 include servers 116 and vendor application database 118 (Fig. 2) similar to the EHR provider 108.

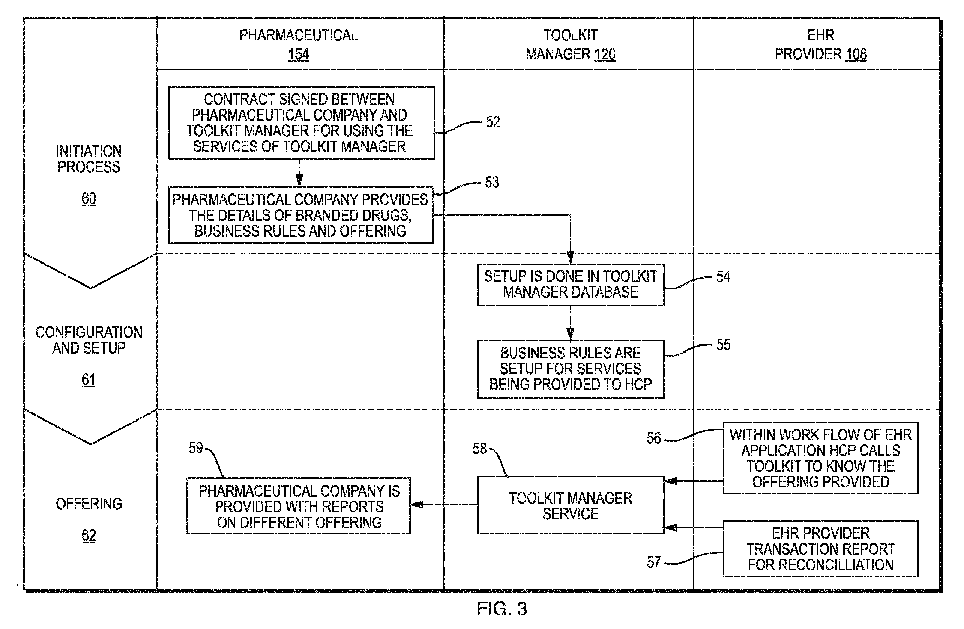

Referring to Fig 3, a workflow block diagram shows the pharmaceutical 154, toolkit manager 120 and EHR provider 108 Steps during the workflow initiation process, configuration and setup, and offerings. The pharmaceutical 154 performs the initiation process 60 in Step 52 by signing a contract for using the service of the tool manager 120. In the next Step 53 pharmaceutical company provides the details of branded drugs, business rules and offerings. Next, a configuration and set up 61 occurs in the Step 54 which performs a set-up in the toolkit database 152. Next, in Step 55 business rules are set up for services provided to the HCP 104. Next, an offering 62 is made where in Step 56 within the EHR application, the HCP calls the toolkit manager 120 to know the offering provided. Further, in Step 57 an EHR provider transaction report for reconciliation is sent to the toolkit manager 120 and in Step 59 pharmaceutical company is provided with reports on different offerings from the Toolkit manager service in Step 58.

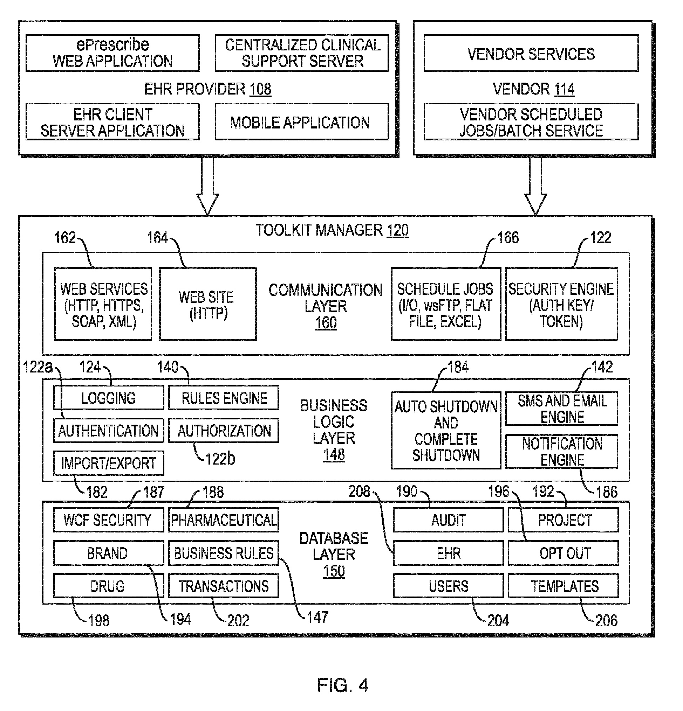

Referring to Fig. 4, Fig. 4 is a functional block diagram of the toolkit manager 120 showing a functional organization of a communication layer 160, a business logic layer 148 and a database layer 150. The communication layer 160 includes web services (HTTP, HTTPS, SOAP and XML) 162, website (HTTP) 164, schedule jobs 166 and security engine 122.

The business logic layer 148 includes logging 124 to logging service request/response and logging application exceptions, and authentication 122a and authorization 122b (governed by security engine 122). Also, import/export 182 is governed by schedule jobs 166 which is used for import/export transaction (done between toolkit manager 120 and EHR) files provided by EHR provider 108, rules engine 140 is used to create business rule and execute in order to validate eligibility criteria of offers; in addition, auto shutdown and complete shutdown 184, SMS and email engine 142 performs two tasks: one is to send coupon link in SMS via Message media service and another is to send coupon/link of coupon by email to patient, and notification engine 186 is to raise notification about any job/service process that has been success/failure.

The database layer 150 has entities which are used to add/update/validate records in database 152. These entities are pharmaceutical 188, audit 190, project 192, brand 194, business rules 147, opt out 196, drug 198, transactions 202, users 204, and templates 206.

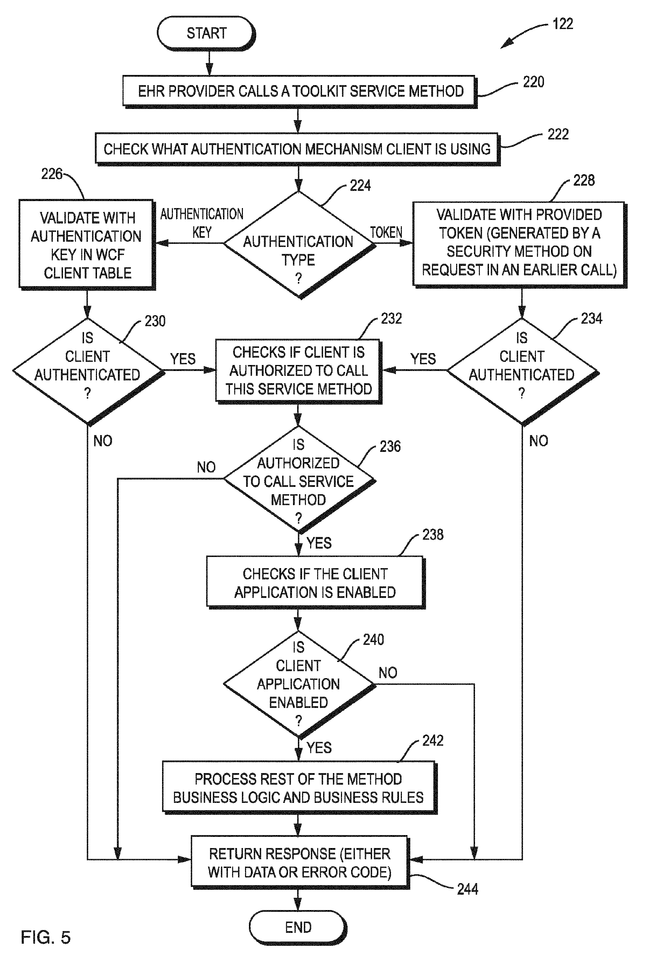

Referring to Fig. 2, Fig. 4 and Fig. 5, security engine 122 is implemented in the toolkit manager 120 in such a way that each call to its service methods gets validated before providing any response back. Each service method call gets authenticated and authorized as shown in the flow chart of Fig. 5 in the following manner:
Authentication 122a: There are two types of authentication implemented in the toolkit manager 120 as follows: (a) Authorization Key 226 validates if Authentication Key used in a service call is valid; and (b) Token 228 validates if provided a Token (generally by a security method on request in an earlier call).

Authorization 122b: Once the authentication is successful 230,234, system checks for the authorization 236 in the following order: (a) system checks if the Client is active, which is identified by the Authorization Key 236 or Token 238 used during authentication; (b) system checks if the relationship is active between the retrieved ClientID and supplied Client Product ID; and (c) system checks the Version supplied by Client and validates the request parameters according to that Version. If any authorization fails, then proper error message is returned to the consumer of the service.

Referring again to Fig. 4, toolkit logging 124 logs all the errors in the file/database along with sending notification email to a toolkit administration in case of any errors from any toolkit components. For Toolkit error logging, Microsoft Enterprise Library's Application Block is used. Microsoft.Practices.Enterprise.Logging Library 124 is used to log the errors in a file and to send the emails to the administrator. By using the Application Block we can ensure that the service is supporting consistency, extensibility, reliability and performance.

Referring to Fig. 2, WCF Services 126 expose different operations methods which authorize the toolkit manager consumer to easily communicate with the toolkit manager 120. Toolkit services are written using .net framework 4.0 technologies with Windows Communication Foundation (WCF) to interact with client. WCF uses service-oriented architecture principles to support distributed computing where services have remote consumers. Clients can consume multiple services; services can be consumed by multiple clients. Services are loosely coupled to each other. All communication with the Windows Communication Foundation (WCF) service 126 occurs through the endpoints of the service. WCF service 126 is exposed on two different endpoints and provides clients access to the functionality offered by the service. WCF Service 126 is exposed over http and https end points. To perform all the functionality, WCF services 126 allow extensive control over the messaging functions of an application. Message is data being transmitted between client and Toolkit WCF Service 126.

The toolkit manager 120 can act as a hub and any vendor can create its own services which in turn can consume toolkit manager vendor services 128. This way toolkit manager 120 is not just limited to an EHR provider 108. Any vendor can be a consumer of this system, and in turn can provide the service to the EHR provider 108 and others wherever required.

Still referring to Fig. 2 Windows service 130 is the function responsible for (a) shutting down the offering/programs for a given component based on the target for that particular offering/programs; and (b) reconciliation of the transactions from various sources, which can be used in reporting. Schedule Job imports and/or exports files (flat file, excel etc.) through FTP or SFTP for importing data from different sources (EHR provider or vendor) to reconciliation of transactions and exports the data for different destinations. Batch Service works as a service provider for sending Email and short message service (SMS) to the end user and logs any request for opting out for any offering/program.

Any vendor 132 can create a scheduled job or batch service which will run nightly/continuously and in turn calls Toolkit WCF Service 126 to get offering/program for that given component.

A business rule engine 140 is used to validate the business rules for each particular program/offering. Core of the business rule engine uses Managed Extensibility Framework (MEF) architecture. A business rule for each program/offering is a loosely coupled component. The Managed Extensibility Framework or MEF is a library for creating lightweight, extensible applications. It allows application developers to discover and use extensions with no configuration required. It also lets extension developers easily encapsulate code and avoid fragile hard dependencies. MEF not only allows extensions to be reused within applications, but across applications as well. The Managed Extensibility Framework (MEF) is a composition layer for .NET that improves the flexibility, maintainability and testability of applications. MEF can be used for third-party plug-in extensibility, or it can bring the benefits of a loosely-coupled plug-in-like architecture to regular applications.

The business rule engine 140 checks if any eligibility check for the offering/programming for that given component needs to be performed before returning the response to the consumer. It loads the required Business Rule libraries at runtime and performs eligibility check and returns the response accordingly. The benefit is that no code changes are required to associate any business rule for performing eligibility check for that offering/program.

The toolkit manager 120 has the ability to create dynamic eligibility business rules with any condition and provide flexibility to a toolkit component to leverage its usage to validate the eligibility business rules by asserting the parameters and its threshold values. This is a robust solution where an Administrator has the ability to configure the eligibility business rules for any component by creating lightweight, extensible extension process code. It lets developers easily encapsulate code and avoid fragile hard dependencies. Unique features of a loosely-coupled plug-in-like architecture for this business rules engine 140 include scalability, extendibility, limited or no knowledge required to set up and integrate business rules, and business rules are exposed as IN Process (business logic library) OR OUT Process (stored procedure and package in database) at runtime without restarting the toolkit manager 120 solution.

An SMS and Email Engine 142 sends SMS and Email to the end user as per the delivery mode selected by EHR provider 108 during prescription (Email, Text) and maintains the delivery status in the database 152. This component internally encapsulates the features of MessageMedia, a third party SMS service provider that sends out SMS. However, other third party SMS providers can be used such as ExactTarget and Constant Contact. Patients can also opt out from the Email and/or SMS, and it is tracked per program/offering. It means that if a patient has opted out for one offering/program for a given component, he/she can receive the text for another offering/program.

Toolkit manager 120 has many components which gives the value added to the prescribers and patients, like electronic coupons 20, medication messaging 22, Adherence 24, etc. and these components are based on service oriented architecture. This gives the flexibility that any consumer can use it with very little effort at the consumer end.

Common entity 146 which are common among all components 144 come into this and they include Brands, Drug, EHR Partner Setup, and Client. For each offering/programs that are created for any component, it belongs to a particular set of drugs. Each drug belongs to particular brand. Drug and brand setup is core to Toolkit components and to allow the scalability across components of toolkit brand and drug setup is made part of common entities.

Referring to Fig. 2 WCF services 126 uses the business logic layer (BLL) 148 for authentication, authorization, validation, business rule evaluation etc. Every type of business logic is written in this layer, and it uses a database layer (DAL) 150 for any database operation.

The database layer (DAL) 150 is the database model of toolkit database 152. This component uses Entity Framework Model 2.0 of Microsoft to interact with the toolkit database 152. The Entity Framework is a set of technologies in ADO.NET that support the development of data-oriented software applications. Architects and developers of data-oriented applications have struggled with the need to achieve two very different objectives. They must model the entities, relationships, and logic of the business problems they are solving, and they must also work with the data engines used to store and retrieve the data. The data may span multiple storage systems, each with its own protocols; even applications that work with a single storage system must balance the requirements of the storage system against the requirements of writing efficient and maintainable application code.

All data flow from different components 144 to the database 152 is carried out through the Entity Framework (EF) 151 Model. This model supports data-centric applications, services and provides a platform for communication to data that raises the level of abstraction from the logical relational level to the conceptual level. The toolkit database 152 uses Microsoft SQL server 2008 R2 for the backend storage of the toolkit manager 120. Microsoft SQL Server 2008 R2 expands on the value delivered in SQL Server 2008 with new technologies and capabilities specifically designed to make actionable intelligence accessible to all employees in the enterprise. It delivers a comprehensive data platform that provides built-in security, availability, and scale coupled with robust business intelligence offerings-helping enable high service levels for mission-critical workloads. A CDC feature (Change Data Capture) is used for capturing any change in data so that a complete history is provided for any changes made in data.

Tables are provided in the database 152 in the following groups: 1) Master: Tables which are having master or supporting data lie in this group; 2) Toolkit: Tables related to all components of the toolkit manager 120 are grouped here; 3) ASP.NET Membership: ASP.Net Membership provider is used to maintain the user and role, so all the tables related to it lie in this group; 4) WCF Tables: All tables which belong to the WCF services 126; and 5) Components: Individual component tables are grouped here.

Referring to Fig. 2 and Fig. 4, Fig. 4 is a more detailed functional design block diagram of the toolkit manager 120 of the present invention. The toolkit manager 120 is functionally organized into a communication layer 160, business layer 148 and database layer 150. Fig. 2 shows the functions performed in each of the functional layers.

The EHR provider 108 is an evolving concept defined as a systematic collection of electronic health information about individual patients or populations. It is a record in digital format that is theoretically capable of being shared across different healthcare settings. In some cases this sharing can occur by way of network-connected, enterprise-wide information systems and other information networks or exchanges. EHRs may include a range of data, including demographics, medical history, medication and allergies, immunization status, laboratory test results, radiology images, vital signs, personal statistics like age and weight, and billing information. EHR applications 105 (web, mobile and desktop) can communicate with Toolkit's WCF Services 126 as the toolkit manager 120 is built on service oriented architecture.

The business flow between the EHR provider 108 and toolkit manager 120 is as follows: (1) After Initial Setup has been completed and a toolkit component has gone live, EHR Application 105 will be calling the web services of different components of toolkit manager 120 to use the service within the prescriber workflow; (2) The HCP while searching for any drug within flow of EHR application 105 will be calling the toolkit service with secure key along with the prescriber and patient parameters; (3) Based on configuration of the service offered to the prescriber 104, eligibility check and business rules are executed by toolkit manager 120 for determining whether to provide the content OR offer to the prescriber or not; (4) Transaction is recorded at toolkit manager 120 for audit trail along with request and response parameters within benchmark time limit; (5) Toolkit manager 120 provides the return result set with possible failure or success message back to the EHR Provider; and (6) Toolkit manager 120 is responsible for receiving the confirmation or cancelation from the EHR application 105 for the status of the transaction that was generated by the EHR application 105 with a request for a given content or offer.

Auto shutdown and complete shutdown 184 is responsible for shutting down the offering/program for a given component in case targets are met through constant monitoring. Once an offer/program is shutdown an email goes to an administrator, who can make it live again or can shutdown completely.

An import/export 182 component is used to import transaction data from excel/flat file (pipe delimited.txt file) and update it in the database 152, which is further used in reconciliation of the transactions generated by the EHR provider 108. The key reason to receive the transaction data back from the EHR provider 108 is to reconcile any open transactions for which the EHR provider 108 was not able to send confirmation or cancelation status. In a similar way data from the system can be exported into the flat file (pipe delimited.txt file) and provided to its vendors on request so that the vendor can generate accurate and robust reporting.

A notification engine 186 is responsible for sending email notifications to the administrator if auto shutdown 184 or threshold conditions are met for a program/offering for a given toolkit component 144.

Pharmaceuticals 188 promote their drugs and provide vital information to both the prescriber 104 and patient 102 in the marketplace through introduction of offerings/programs for a given toolkit component 144 like medication messaging component 22, electronic coupon component 20 offers, patient education material or patient adherence component 24 programs from the EHR provider 108 within an ePrescription and EHR application 105.

Toolkit manager 120 generates a new Client ID for each new pharmaceutical company and collects their details. These details are captured only for the purpose of printing reports and linking drug(s) to offering/programs for any given component to the pharmaceutical company. A pharmaceutical brand manager has the capability to view the reports using Admin Tool 156.

An auditing 190 capability included in the toolkit manager 120 provides ability to record failed authentication attempts, tracking of IP address for the source of all requesting WCF service 126 calls, not recording identifiable patient data elements in the audit trail, tracking all the input and output parameters passed to them WCF service 126 calls by the EHR provider 108, and recording and maintaining changes to all the key data elements such as brand name, drug name, individual drug information like NDC, RxNorm(s), Offer/Program information, Offer/Program start date, Offer/Program end date, Offer/Program is assigned to which drugs and EHRs.

Still referring to Fig. 4, a project 192 is an engagement made with a pharmaceutical client (which is a legal relationship with a client to deliver a contracted functionality) by signing a statement of work, and a Prescriber Toolkit Project Manager creates a new project 192 in toolkit manager 120 which consists of a budget for the project, brand associated with it, start and end dates of the project for the particular client and brand(s). One project 192 can be linked to a single pharmaceutical client.

A brand 194 refers to the brand name of the pharmaceutical drugs, which have association with the toolkit manager 120.

The business rules 147 relate to each offering/ program for any given toolkit component 144 and it can have different business rules for the eligibility checking, so these are the entities which check for the actual business rule associated with a particular offering/program.

An opt out 196 function provides the ability to unsubscribe patient and/or physician who receives email and SMS communications from the system at different levels. Physician and/or patient can be unsubscribed at the offering level, campaign level, brand level or pharmaceutical client level.

A drug entity 198 records the drug information and associate to its pharmaceutical client. These drugs are attached to the offering/program for any given component provided during initiation process of that program. Once new drug information has been added in toolkit manager 120, a new toolkit Unique Drug is generated which is provided in Initiation form to EHR partners/vendors.

Each valid request for any offering/program for any given component is logged as a transaction 202 and its transaction ID is used for any further communication for that particular transaction.

Users 204 are the toolkit manager 120 Admin Tool 156 (Fig. 1) users who have roles and responsibilities regarding setup offering/program for any given toolkit component 144, analyzing reports, setup import/export feature, communication with vendors/EHR provider. User entity maintains the detail for toolkit manager 120 users and their roles.

The toolkit manager 120 uses a template 206 for offering/program for any given component or text template for sending email/SMS to the patients. Each vendor 114 can have their own different templates, so this component contains information about the respective vendor templates in toolkit manager 120. These templates are further used by a PDF & Imager Generator component to generate in PDF/JPG format and stored on the file system to facilitate the generation of materials for any component of toolkit manager 120.

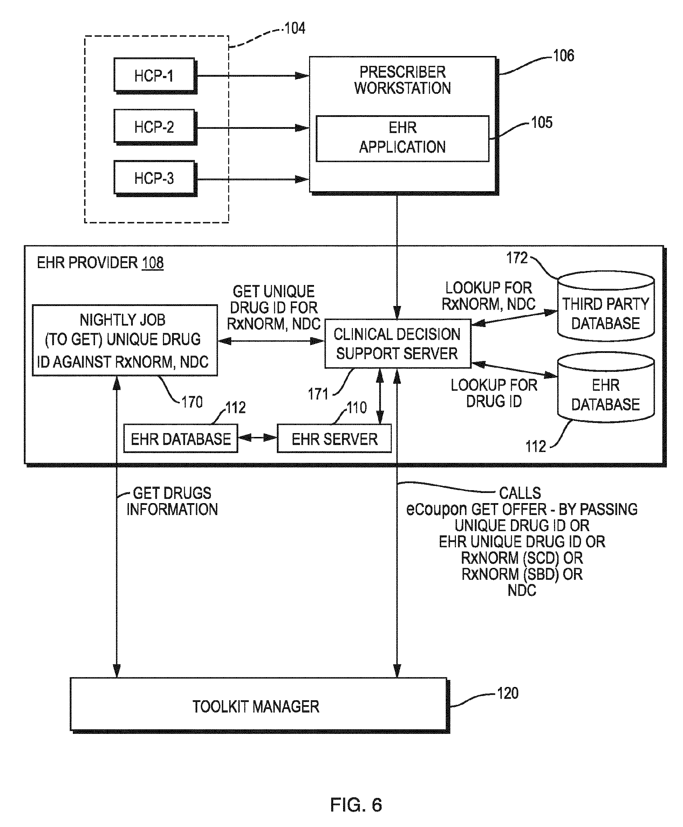

Referring to Fig. 6, Fig. 6 is a block diagram of a drug setup process using a unique drug identifier. A Unique Drug ID is generated by toolkit manager 120 for each drug (for specific Name/Strength/Unit/Route/Form). This is provided at a time of initiation process from toolkit manager 120 to the EHR provider 108. This ID is unique and will be the same during the lifecycle of the toolkit component 144 for specific Name/Strength/Unit/Route/Form.

The unique drug identifier is the key factor which will be used between EHR provider 108 and toolkit manager 120. Toolkit manager 120 will have the capability to provide and configure either of the following type of Drug Identifier for communication with EHR providers 108: (a) Toolkit Unique Drug ID (see Table 1); (b) Rx Norm ID-Semantic Branded Drug (see Table 2); (c) Rx Norm ID-Semantic Clinical Drug (see Table 3); (d) EHR Unique Drug ID (See Table 4), and (e) NDC (National Drug code) (See Table 5).

**TABLE 1 Unique Drug ID**

| TM Drug ID | Drug Name | **Unique Drug ID [Primary Key in Toolkit]** | Strength | Unit | Dosage Form | Route |
|---|---|---|---|---|---|---|
| 501 | Lipitor | **1001** | 10 | MG | Tablet | Oral |
| | | **1002** | 20 | MG | Tablet | Oral |
| | | **1003** | 40 | MG | Tablet | Oral |
| | | **1004** | 80 | MG | Tablet | Oral |

**TABLE 2 RxNorm ID (SBD)**

| TM Drug ID | Drug Name | **RxNorm Semantic Branded Drug ID [Mapped to Unique Drug ID]** | Strength | Unit | Dosage Form | Route |
|---|---|---|---|---|---|---|
| 501 | Lipitor | **617314** | 10 | MG | Tablet | Oral |
| | | **617318** | 20 | MG | Tablet | Oral |
| | | **617320** | 40 | MG | Tablet | Oral |
| | | **29205** | 80 | MG | Tablet | Oral |

**TABLE 3 RxNorm ID (SCD)**

| TM Drug ID | Drug Name | **RxNorm Semantic Clinical Drug ID [Mapped to Unique Drug ID]** | Strength | Unit | Dosage Form | Route |
|---|---|---|---|---|---|---|
| 501 | Lipitor | **617312** | 10 | MG | Tablet | Oral |
| | | **617310** | 20 | MG | Tablet | Oral |
| | | **617311** | 40 | MG | Tablet | Oral |
| | | **259255** | 80 | MG | Tablet | Oral |

**TABLE 4 EHR Unique Drug ID**

| TM Drug ID | Drug Name | **EHR Application Unique Drug ID [Mapped to Unique Drug ID]** | Strength | Unit | Dosage Form | Route |
|---|---|---|---|---|---|---|
| 501 | Lipitor | **6200** | 10 | MG | Tablet | Oral |
| | | **5600** | 20 | MG | Tablet | Oral |
| | | **8500** | 40 | MG | Tablet | Oral |
| | | **1700** | 80 | MG | Tablet | Oral |

**TABLE 5 NDC (National Drug Code) is mapped to Unique Drug ID representing unique drug identifier**

| TM Drug ID | Drug Name | Strength | Unit | Dosage Form | Route | Packaging | **NDC [Mapped to Unique Drug ID]** |
|---|---|---|---|---|---|---|---|
| 501 | Lipitor | 10 | MG | Tablet | Oral | 90 EA Bottle | **71015523** |
| | | | | | | 5000 EA Bottle | **71015534** |
| | | | | | | 100 EA box | **71015540** |
| | | 20 | MG | Tablet | Oral | 90 EA Bottle | **71015623** |
| | | | | | | 100 EA box | **71015640** |
| | | | | | | 5000 EA Bottle | **71015694** |
| | | 40 | MG | Tablet | Oral | 90 EA Bottle | **71015723** |
| | | | | | | 100 EA box | **71015740** |
| | | | | | | 5000 EA Bottle | **71015773** |
| | | 80 | MG | Tablet | Oral | 90 EA Bottle | **71015823** |
| | | | | | | 500 EA Bottle | **71015873** |
| | | | | | | 2500 EA Bottle | **71015888** |
| | | | | | | 64 EA Box | **71015892** |

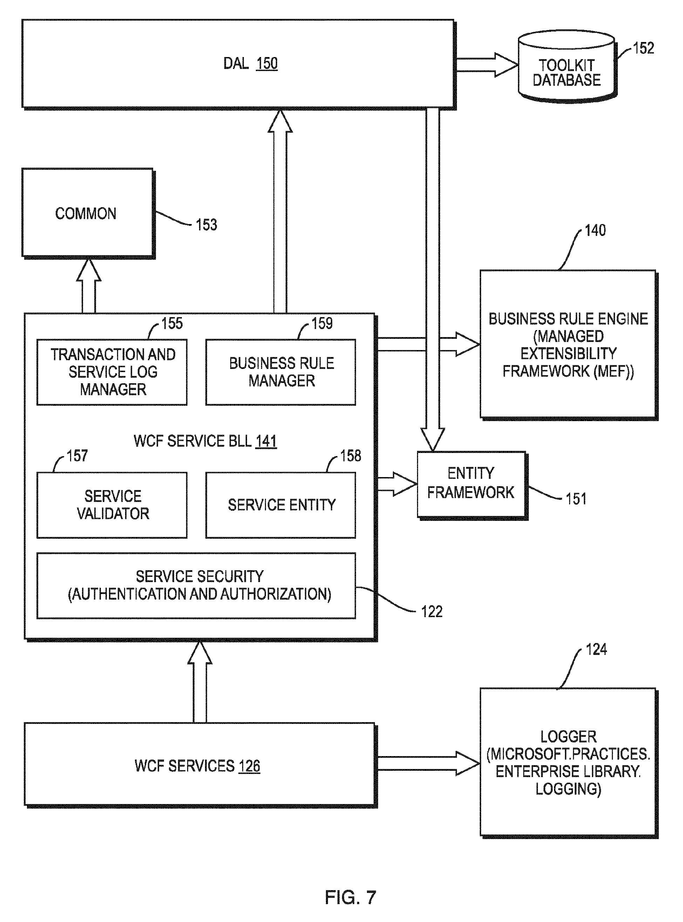

Referring to Fig. 7, Fig. 7 shows a block diagram of windows communication foundation (WCF) service 126 operation interfacing with the toolkit manager database 152, business rules engine 140 and logger 124. The communication between the EHR provider 108 and the toolkit manager 120 uses a common set of methods.

The common four general methods that exists in the toolkit manager 120 for each of the toolkit components 144 are as follows: (1) The First method is to check if the offering/ program for a given component is available and return the details of the offering, if available, as the response to a request; (2) The Second method is used to confirm that the provider is consuming/displaying or using the offering/program by sending the confirmation call back for the transaction ID returned in the first method call; (3) The Third method is used to cancel if EHR provider 108 does not want to provide the offering/program provided by the toolkit manager 120 by sending the cancellation call for the transaction ID returned in the first method call; and (4) The Fourth Method can be used by the nightly job that is created on the EHR provider side to retrieve all the offerings/programs provided by the toolkit manager 120 for that client. Client can use the response to setup details of the offering/programs in their system if required.

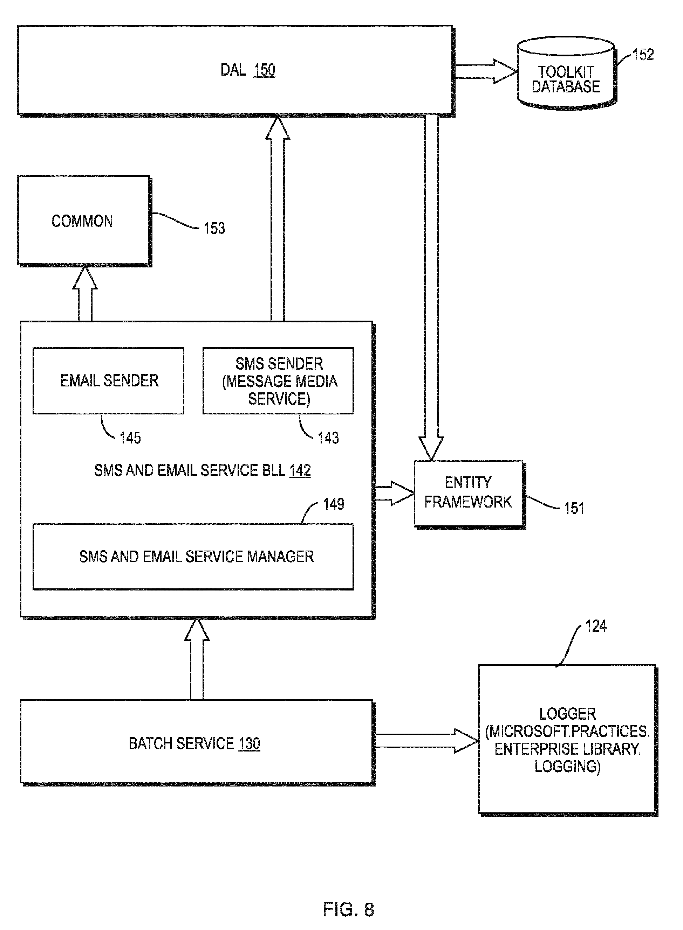

Still referring to Fig. 7, WCF Service business logic layer (BLL) 141 contains all internal business rules in an abstract manner. It acts as an intermediate actor between WCF service 126 and Entity Framework (EF) 151, which receives the call from WCF services 126 and passes the data further to the database layer (DAL) 150 after validating the data to fulfill the requirement. Entity Framework (EF) 151 is an object-relational mapper that enables .NET developers to work with relational data using domain-specific objects in application code. It eliminates the need for most of the data-access code that developers usually need to write. Common 153 is part of Common entity 146 (Fig. 2) which are common among all components 144 within Toolkit Manager 120, and they include Brands 194, Drug 198, EHR provider Setup, and Client. Common 153 is a bundle of common functions and methods that are required by Service validator 157, Email sender 145 and Service security 122 so it is shown as a separate box in both Figure 7 and Figure 8. For each offering/programs that are created for any component, it belongs to a particular set of drugs. Each drug belongs to particular brand. Drug and brand setup is core to toolkit components and to allow the scalability across components of toolkit brand and drug setup is made part of common entities. WCF Service BLL 141 needs to access all the Common entities 146 to look-up client, brand, drug and unique drug information.

Transaction and service log manager 155 is responsible to communicate to database 152 via DAL 150 (Database Layer) for creation and maintenance of a transaction for each request for any of the toolkit components 144. It is also responsible for maintenance of WCF service logs for each and every request made to WCF services 126. Transaction and service log manager 155 is responsible for creation of the transaction identifier for each request and updates the transaction status for that particular transaction identifier based on the response received from Business Rule Manager 159, Service Validator 157 and Service Security 122.

Service Validator 157 is responsible for checking if the WCF request/call made by the EHR provider 108 is valid or invalid for that particular service. Service validator 157 performs following checks to make sure that the requires service request call is valid: (a) Service and Service Method requested exists and is assigned to that particular EHR; (b)Request parameters passed in the service method are all valid; and (c) Service and Service Method is up and running and is not inactivated or disabled. Service Entity 158 contains C SHARP (C#) classes (programming language for Microsoft .Net Framework) for all the WCF Table structure created using entity framework 151. Service Entity 158 is used by Service Validator 157 to talk to database 152 and validate if the given service and service method is assigned to particular EHR provider 108 client.

Common entity 146 (Fig. 2) are used by WCF Services 126. Client can communicate with WCF service 126 and the data contract for communications is defined as part of WCF services 126. Common entity 146 implements data contract and describes the external format of data passed to and from WCF service 126 operations. This is a formal agreement between the toolkit manager 120 service and a client that abstractly describes the data to be exchanged. That is, to communicate, the client and the WCF service 126 do not have to share the same types, only the same data contracts. A data contract precisely defines the data type, and if the parameter is required or not, for each input/output parameter.

Toolkit manager 120 has a security 122 subsystem which validates the WCF service 126 call for authentication and authorization. The toolkit manager 120 contains separate modules to handle all the activities related to an offering/programs, transaction and error logging as well as business rule validation through business rule engine 140 or stored procedure according to the business rule type defined database.

Referring to Fig. 8, Fig. 8 is a block diagram of a batch service operation interfacing with the toolkit database 152, entity framework 151, logger 124 and SMS and email services business logic level 142. Entity Framework 151 is an object-relational mapper that enables .NET developers to work with relational data using domain-specific objects in application code. It eliminates the need for most of the data-access code that developers usually need to write. The block diagram in Fig. 8 shows the batch service 130 which communicates to SMS and Email Service business logic layer 142 (BLL) component to fetch data from database 152 and sends SMS and Email to the end user as per the delivery type (Email, Text) preferences stored in database 152 as well as update the same delivery status in a database table. This SMS and Email service BLL 142 module internally encapsulates the feature of a "messagemedia service" 143 to send SMS and Email sender subcomponent for Emailing. The Batch Service 130 process runs in a particular interval of time span which is defined in an application configuration file to send out email and SMS. In the present embodiment it is set to run every 300 seconds.

Toolkit manager 120 has the ability to create dynamic eligibility business rules with any condition and provide flexibility to a toolkit component to leverage its usage to validate the eligibility business rules by asserting the parameters and related threshold values. This provides the Administrator with the ability to configure the eligibility business rules for any components by creating lightweight, extensible extension process code. It lets developers easily encapsulate code and avoid fragile hard dependencies.

The following are unique features of loosely-coupled plugin-like architecture for Business Rules Engine 140: scalability; extendibility; limited or no knowledge required to setup and integrate business rules; Business Rules are exposed as IN Process (business logic library) OR OUT Process (stored procedure and package in database) at runtime without restarting the toolkit manager 120.

Examples of different type parameters and associated operators, which can be used with the setup of business rules, are shown in Table 6.

**TABLE 6**

| **Category** | **Attribute/ Parameter** | **Operator** | **Threshold value type** | **Notes** |
|---|---|---|---|---|
| BR-1 : Provider | NPI Number | EQUAL | Alpa Numeric | Multiple values |
| BR-2 : Provider | NPI Number | NOT EQUAL | Alpa Numeric | Multiple values |
| BR-3 : Provider | Specialty Code | EQUAL | Alpa | Multiple values |
| BR-4 : Provider | Specialty Code | NOT EQUAL | Alpa | Multiple values |
| BR-5 : Provider | State Code | EQUAL | Alpa | Multiple values |
| BR-6 : Provider | State Code | NOT EQUAL | Alpa | Multiple values |
| BR-7 : Patient | Age | LESS THAN | Numeric | Single Value |
| BR-8 : Patient | Age | GREATER THAN | Numeric | Single Value |
| BR-9 : Patient | Gender | EQUAL | Alpa | Single Value |
| BR-10 : Provider | LastPrescribedDate | LESS THAN | DateTime | Single Value |
| BR-11 : Provider | LastPrescribedDate | NOT EQUAL | DateTime | Single Value |
| BR-12 : Provider | Practice Zip | EQUAL | Numeric | Single Value |
| BR-13 : Provider | Practice Zip | NOT EQUAL | Numeric | Single Value |

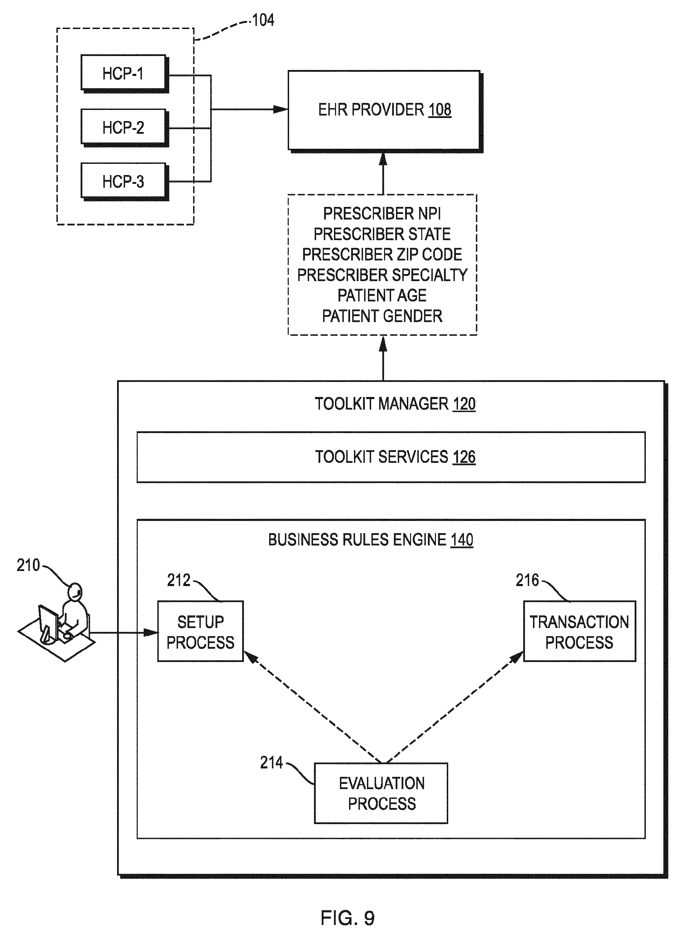

Referring now to Fig. 9, Fig. 9 is a flow chart of the business flow between an EHR provider 108 and the toolkit manager 120, and it illustrates a method for setting up business rules as follows: (1) An Operation team 210 first sets up the business rules in the toolkit manager 120 for the product/component for a particular drug based on various offerings agreed with a Pharmaceutical 154 provided to a patient through the EHR provider 108; (2) The EHR provider 108 using the EHR application 105 will pass patient and prescriber parameters values through the security 122 layer to Toolkit Service 126 for evaluating whether subscribed product is successfully eligible to get the offer/content/program or not; and (3) Toolkit manager 120 calls business rules engine 140 to evaluate during an evaluation process 214 the eligible criteria for given parameters and transaction 216 and whether the process is success or failed based on logic setup 212 configured by the operation team 210.

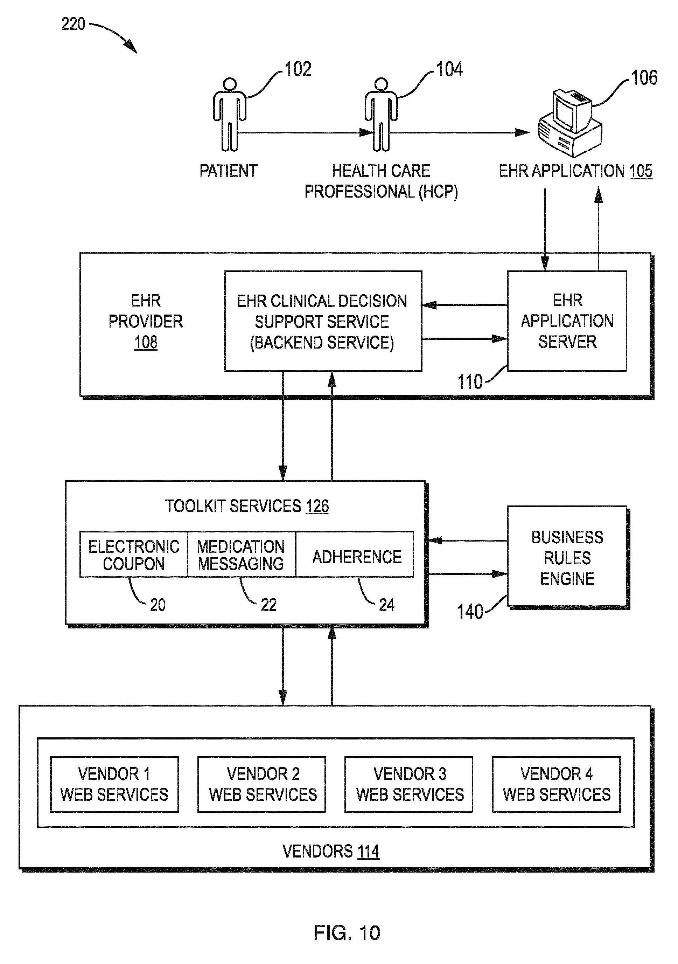

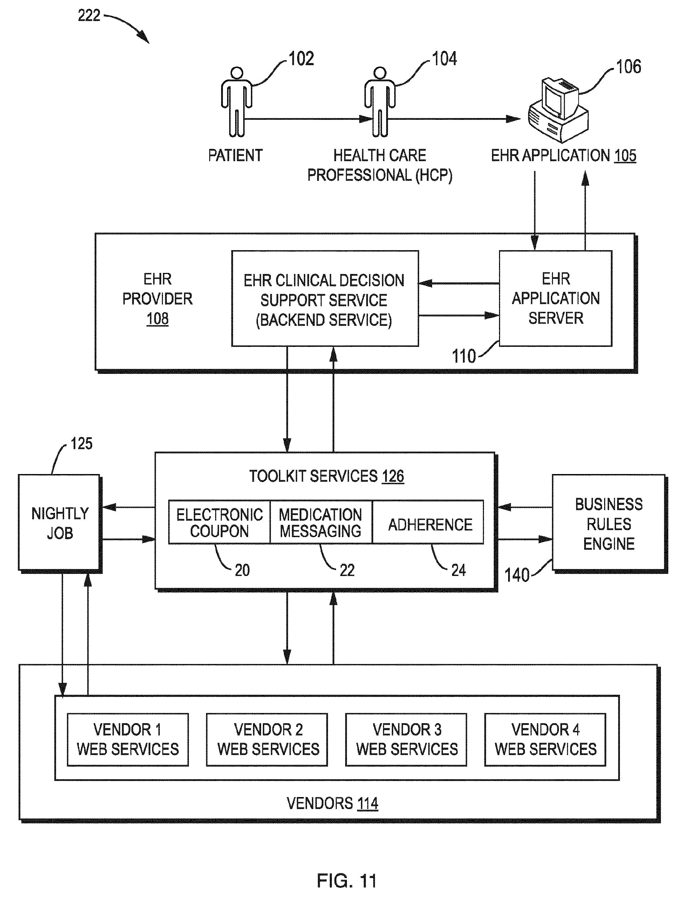

Referring to Figs. 10 and 11, the toolkit manager 120 can function as a HUB. In the HUB 220 embodiment shown in Fig. 10, the EHR provider 108 makes calls to toolkit WCF services 126. EHR provider 108 does not need any other implementation to communicate to external third party vendors. The toolkit manager 120 acts as a HUB and translates calls from EHR provider 108 to each individual third party vendor based on a vendor agreement. In the second HUB 222 embodiment shown in Fig. 11, nightly job 125 runs on toolkit manager 120 or third party vendors 114 to push or pull configuration/setup into the Toolkit Business Rules Engine 140. The advantage of this embodiment will be quick response time to EHR provider 108 calls.

This invention has been disclosed in terms of a particular embodiment. It will be apparent that many modifications can be made to the disclosed apparatus and method without departing from the invention. Therefore, it is the intent of the appended claims to cover all such variations and modifications as come within the true spirit and scope of this invention.

## Claims

1. A toolkit manager system for managing delivery of services and content to a prescribing healthcare professional via an electronic health record provider comprising:
a server computing device including a memory and a processor;
a database connected to said server computing device having stored therein pharmaceutical drug data and business rules;
communication services located within said server computing device for interfacing with said electronic health record provider via web internet services;
security means for providing authentication and authorization to verify that calls are received from said electronic health record provider that are allowed access;
said server computing device verifies eligibility of a patient and said healthcare professional for a toolkit service using said business rules data stored in said database; and
said server computing device comprises executable instructions stored in said memory which when executed by the processor selects said toolkit services to be sent by said communication services to said electronic health record provider and said healthcare professional.

2. The toolkit manager system as recited in Claim 1 wherein said system comprises a toolkit manager for controlling access to services including electronic coupon, medication messaging and patient adherence, said toolkit manager including said server computing device and said database.

3. The toolkit manager system as recited in Claim 1 wherein said system comprises a toolkit manager having an input for receiving into said database pharmaceutical offerings on drugs.

4. The toolkit manager system as recited in Claim 1 wherein said server computing device generates a unique drug ID for each drug communicated with said electronic health record provider.

5. A computer-implemented method for managing delivery of services and content to a prescribing healthcare professional via an electronic health record provider comprising:
providing a server computing device including a memory and a processor;
storing pharmaceutical drug and business rules in a database connected to said server computing device;
providing communication services located within said server computing device for interfacing with said electronic health record provider via web internet services;
performing authentication and authorization of said electronic health record provider via said web internet services;
verifying eligibility of a patient and said healthcare professional for a toolkit service with said server computing device using said business rules data stored in said database; and
providing executable instructions stored in said memory of said server computing device which when executed by said processor selects said toolkit service to be sent by said communication services to said electronic health record provider and said healthcare professional.

6. The method as recited in Claim 5 wherein said method comprises the step of controlling access to services including electronic coupon, medication messaging and patient adherence using a toolkit manager having said server computing device and said database.

7. The method as recited in Claim 5 wherein said method comprises the step of providing said toolkit manager with an input for receiving pharmaceutical offerings/programs on drugs into said database.

8. The method as recited in Claim 5 wherein said method comprises the step of generating a unique drug ID by said server computing device for each drug communicated with said electronic health record provider.

9. A non-transitory computer-readable medium having computer-executable instructions for performing a computer-implemented method for managing delivery of services and content to a prescribing healthcare professional via an electronic health record provider comprising:
providing a server computing device including a memory and a processor;
storing pharmaceutical drug and business rules in a database connected to a server computing device having a memory and a processor;
providing communication network located within said server computing device for interfacing with said electronic health record provider via web internet services;
performing authentication and authorization of said electronic health record provider via said web internet services;
verifying eligibility of a patient and said healthcare professional for a toolkit service with said server computing device using said business rules data stored in said database; and
providing executable instructions stored in said memory of said server computing device which when executed by said processor selects said toolkit service to be sent by said communication network to said electronic health record provider and said healthcare professional.

10. The computer-readable medium as recited in Claim 9 wherein said method comprises the step of controlling access to services including electronic coupon, medication messaging and patient adherence using a toolkit manager having said server computing device and said database.

11. The computer-readable medium as recited in Claim 9 wherein said method comprises the step of providing said toolkit manager with an input for receiving pharmaceutical offerings/programs on drugs into said database.

12. The computer-readable medium as recited in Claim 9 wherein said method comprises the step of generating a unique drug ID by said server computing device for each drug communicated with said electronic health record provider.
